# EUROPÄISCHE PATENTSCHRIFT

(11) **EP 0 505 966 B1**
(45) Veröffentlichungstag und Bekanntmachung des Hinweises auf die Patenterteilung: **28.06.1995**
(21) Anmeldenummer: 92104995.3
(22) Anmeldetag: 23.03.1992
(51) Int. Cl.: A61K 9/16

(54) **Langwirkende bioabbaubare Mikropartikel und ein Verfahren zur Herstellung**
Long-acting biodegradable microparticles and process for preparation
Microparticules biodégradables à effet prolongé et procédé de préparation

(30) Priorität: 25.03.1991 DE 4109746
(43) Veröffentlichungstag der Anmeldung: 30.09.1992
(73) Patentinhaber: HOECHST AKTIENGESELLSCHAFT, 65926 Frankfurt am Main (DE)
(72) Erfinder: Lill, Norbert, Dr., W-6242 Kronberg/Taunus (DE); Sandow, Jürgen, Dr., W-6240 Königstein/Taunus 3 (DE)

(56) Entgegenhaltungen:
- EP-A- 0 257 915
- EP-A- 0 315 875
- FR-A- 2 620 621
- FR-A- 2 649 319
- DIE PHARMAZIE Bd. 47, Nr. 2, Februar 1992, FRANKFURT/MAIN Seiten 115 - 119;
- THOMA K. ET AL: 'BEZIEHUNGEN ZWISCHEN HERSTELLUNGESPARAMETERN UND PHARMAZEUTISCH-TECHNOLOGISCHEN ANFORDERUNGEN AN BIODEGRADIERBARE MIKROPARTIKELN'
- BIOMATERIALS Bd. 11, November 1990, GUILFORD,SURREY,GB Seiten 679 - 685; WANGH.T. ET AL: 'DEGRADATION OF POLYESTER MICROSPHERES'

## Beschreibung

Die Erfindung betrifft langwirkende, biologisch abbaubare Mikropartikel auf Basis von Poly(lactid-co-glycolid) (=PLGA), die therapeutisch wirksame Peptide bzw. deren physiologisch verträgliche Salze als Wirkstoff enthalten, sowie ein Verfahren zur Herstellung solcher Mikropartikel.

Für Arzneistoffe, die im Gastrointestinaltrakt enzymatisch zerstört werden, wie z. B. Proteine oder Peptide, sind neben einer nasalen und dermalen Applikation - mit oft nur geringer Resorptionsrate - vor allem parenterale Arzneiformen von Bedeutung. Eine Langzeitbehandlung bestimmter Krankheiten läßt es wünschenswert erscheinen, parenterale Depotarzneiformen zu entwickeln, die den Arzneistoff kontinuierlich über mehrere Wochen freigeben. In der Literatur werden für diese Zwecke neben Implantaten (vergl. z. B. EP-B-0 058 481) auch Mikrokapseln/Microspheres beschrieben.

Um ein operatives Entfernen dieser Injektionsformen zu vermeiden, verwendet man bioabbaubare Polymere. Neben Polyamiden, Polyanhydriden, Poly(ortho)ester, Polyacetale werden vor allem Polyester eingesetzt. Meist werden Polyester auf Basis der Monomeren Milchsäure und Glykolsäure oder deren Copolymere als geeignete Polymere beschrieben.

Zur Herstellung von Mikrokapseln/Microspheres kommen verschiedene Verfahren, wie z. B. Phasenseparation (EP-B-0 052 510), Solvent extraction (EP-B-0 145 240) oder die Sprühtrocknung (EP-A-0 315 875 = ZA-Patentschrift 88/8396, CH 666 406, A5, vergl. Derwent Ref. Nr. 88-228036/33) in Betracht. Die Sprühtrocknung hat gegenüber den anderen Verfahren den Vorteil, daß die Verkapselungseffizienz in der Regel höher liegt als bei den anderen genannten Verfahren und daß keine weiteren Hilfsstoffe für die Verkapselung benötigt werden, die dann im Produkt zu möglichen Verunreinigungen führen würden. Nachteilig ist, daß die Sprühtrocknung bei höheren Temperaturen, d. h. in der Regel weit über dem Siedepunkt der jeweiligen Lösungsmittel, durchgeführt werden muß. Unterwirft man Suspensionen, Emulsionen oder Lösungen von Copolymeren der Milch- und Glykolsäure (PLGA) unter üblichen Bedingungen der Sprühtrocknung, so erhält man keine Mikropartikel, sondern fadenartige Gebilde, die sich nicht für eine injizierbare Depotform eignen (EP-A-0 315 875). Die in CH 666 406 A5 beschriebenen durch Sprühtrocknung bei 59 °C erhaltenen Mikrokapseln enthalten als biologisch abbaubares Trägermaterial nicht PLGA sondern D, L-Oligolacoyl-N-(L)-Phenylalanin. H.T. Wang et al. (Biomaterials 1990, Band 11, Seiten 679-685) beschreiben Mikropartikel die Albumin und PLGA enthalten und durch Sprühtrocknung bei etwa 37°C erhalten werden.

Überraschenderweise wurde nun gefunden, daß sich PLGA-Polymerlösungen und entsprechende wäßrige Suspensionen und Emulsionen zu Mikropartikeln versprühen lassen, wenn die Sprühtemperatur weniger als 60 °C und der Sprühflow über 500 NL/h beträgt. Diese Mikropartikel zeichnen sich zudem noch, verglichen mit Mikropartikeln, die nach den anderen erwähnten Verfahren hergestellt werden, durch einen geringeren Restlösungsmittelgehalt (organisches Lösungsmittel, Wasser), der unter 1 % liegt, aus. Letzteres ist insbesondere bei Injektabilia von großer Bedeutung, da hier hohe Anforderungen an die Reinheit eines Arzneimittels gestellt werden.

Die Erfindung betrifft daher Mikropartikel, die ein Peptid als Wirkstoff und Poly(lactid-co-glycolid) (PLGA) als Trägermaterial enthalten, dadurch gekennzeichnet, daß sie durch Sprühtrocknung bei Temperaturen unter 60 °C bei einem Sprühflow über 500 NL/h erhalten werden.
(1 NL bezieht sich auf die Verhältnisse bei einer Temperatur von 0°C und einem Druck von 1013 hPa und entspricht 1 L).

In den vorstehenden und folgenden Ausführungen werden unter Mikropartikel auch Mikrokapseln und Microspheres verstanden, also sowohl Teilchen, bei denen der Wirkstoff vom Polymer vollständig oder teilweise eingeschlossen ist, als auch Teilchen, bei denen der Wirkstoff in einer PLGA-Matrix verteilt ist.

Unter Peptide werden natürliche und synthetische Peptide sowie deren physiologisch verträgliche Salze verstanden.

Die Erfindung betrifft ferner ein Verfahren zur Herstellung von Mikropartikeln, die ein Peptid als Wirkstoff und PLGA als Trägermaterial enthalten, dadurch gekennzeichnet, daß man das Peptid in einer Lösung des Trägermaterials suspendiert oder löst oder eine wäßrige Lösung des Peptids in einer Lösung des Trägermaterials emulgiert und die Suspension, Lösung oder Emulsion bei einer Temperatur unter 60 °C bei einem Sprühflow größer als 500 NL/h versprüht.

Die erfindungsgemäßen Mikropartikel enthalten vorzugsweise wasserlösliche Peptide. Das Molekulargewicht der Peptide liegt vorzugsweise über 800. Besonders geeignete Peptide sind LHRH-Agonisten und -Antagonisten wie z. B. Buserelin, HOE 013 (Ac-D-Nal-p-Cl-D-Phe-D-Trp-Ser-Tyr-D-Ser (α-L-Rha)-Leu-Arg-Pro-Azagly-NH₂, vgl. EP-A-0 263 521), Nafarelin, Triptorelin, Leuprorelin und Goserelin. Peptide anderer Typen sind ebenfalls geeignet wie z. B. TRH, Vasopressin, Calcitonin, Insulin oder HOE 427 (=Ebiratide, [4-Methionindioxid, 8-D-Lysin, 9-Phenylamin]-α-MSH-(4-9)-(8-aminooctyl)amid-triacetat, vgl. EP-A-0 179 332).

Das biologisch abbaubare Trägermaterial ist PLGA. Die Wirkstofffreigabe aus den Mikropartikeln wird nicht nur durch die physikalisch-chemischen Eigenschaften des Wirkstoffes sondern auch durch die Eigenschaften des Polymers oder der Polymermischungen wie Molekulargewicht, molare Zusammensetzung, Abfolge der Milchsäure und Glykolsäureeinheiten im Polymer beeinflußt. Das Trägermaterial kann z. B. aus einem Gemisch von PLGA 50 : 50 und PLGA 40 : 60 bestehen. Je höher das Molekulargewicht bzw. die inhärente Viskosität ist, desto länger hält die Wirkstofffreigabe an. Die inhärente Viskosität (20 °C, Chloroform, 0,1 %) beträgt vorzugsweise 0,1 bis 0,8 dl/g. Das Molverhältnis von Lactid zu Glykolideinheiten liegt im Bereich z. B. von 85 : 15 bis 40 : 60, vorzugsweise wird Poly(d,l-lactid-co-glycolid) 50 : 50 verwendet, das eine Viskosität von 0,1 bis 0,7 dl/g, insbesondere von 0,1 bis 0,5 dl/g, aufweist.

Je nach Eigenschaften des Polymers geben die nach dem erfindungsgemäßen Verfahren hergestellten Mikropartikel den Wirkstoff über einen Zeitraum von vorzugsweise 2 Wochen bis 3 Monaten frei. Der Beladungsgrad hat ebenfalls einen, wenn auch untergeordneten, Einfluß auf die Dauer der Wirkstofffreigabe. Bei dem erfindungsgemäßen Verfahren werden vorzugsweise Mikropartikel mit einem Peptid-Beladungsgrad von weniger als 20 %, vorzugsweise von weniger als 12 % erhalten.

Die Partikelgröße, die ebenfalls die Freigabe beeinflußt, ist bei den nach dem erfindungsgemäßen Verfahren hergestellten Mikropartikeln kleiner als 200 »m, vorzugsweise kleiner als 100 »m. Insbesondere ist die Partikelgröße kleiner als 50 »m.

Die Herstellung der Mikropartikel erfolgt durch Versprühen aus Lösung, Emulsion oder Suspension des Wirkstoffes in der Polymerlösung. Als Lösungsmittel können beispielsweise Chloroform, Methylenchlorid, DMF, Aceton, Essigsäureethylester, Eisessig und Wasser oder deren Gemische eingesetzt werden. Vorzugsweise werden Methylenchlorid, Eisessig und Wasser verwendet.

Bei dem erfindungsgemäßen Verfahren wird das Peptid vorzugsweise in Wasser gelöst und in die Lösung von PLGA in z. B. Methylenchlorid gegeben. Die hierbei entstehende Emulsion wird vorzugsweise versprüht. Die Versprühung kann auch nach Zusatz von z. B. Methanol, wobei dann eine Lösung entsteht, erfolgen.

Die Sprühtemperatur liegt vorzugsweise bei 50 ° bis 30 °C, insbesondere bei 40 ° bis 30 °C. Der Sprühflow darf nicht zu niedrig sein, da es sonst zu Fadenbildung kommen kann. Er beträgt vorzugsweise etwa 800 NL/h, da mit einem höheren Sprühflow bessere Trocknungseigenschaften verbunden sind.

Der Druck des Sprühmediums, beispielsweise Luft oder Stickstoff, sollte bei Verwendung eines Laborsprühgerätes (z. B. Büchi Mini Spray Dryer 190) im Bereich 3 - 8 bar liegen. Die Pumpenleistung des Sprühgerätes muß den Gegebenheiten angepaßt werden (3 - 20 ml/min). Durch entsprechende Einstellung des Aspirators z. B. 18 Skalenteile, wird gewährleistet, daß alle im Sprühmedium befindliche Luft bzw. Stickstoff abgezogen wird.

Das PLGA-Polymer sollte in möglichst wenig Lösungsmittel gelöst werden, wobei Polymere mit hohem Anteil an Laktid eine größere Lösungsmittelmenge erfordern. Prinzipiell ist die Konzentration des Polymers in der zu versprühenden Lösung, Emulsion, Suspension so zu wählen, daß unter den gewählten Bedingungen eine Versprühung möglich ist. Vorzugsweise beträgt die PLGA Konzentration in Methylenchlorid weniger als 15 Gew.-% berechnet auf Methylenchlorid.

Durch die folgenden Beispiele wird die Erfindung erläutert.

Bei der in den Beispielen für PLGA angegebenen Viskosität handelt es sich um die inhärente Viskosität einer 0,1 %igen Lösung in Chloroform bei 20 °C bestimmt nach allgemein üblichen Methoden.

Die Sprühparameter a) Aspirator und b) Pumpenleistung werden bei allen Beispielen auf 18 (für Aspirator) bzw. 6 (für Pumpenleistung) Skalenteile eingestellt.

### Beispiel 1: Sprühmedium - Eisessig

a) 0,064 g Buserelinacetat werden in 2,0 g Wasser gelöst. Die wäßrige Lösung wird mit einer Lösung von 1,936 g PLGA 50:50 (IV = 0,4 dl/g) in 40 g Eisessig gemischt und in einem Laborsprühtrockner versprüht.
Die Sprühparameter werden wie folgt eingestellt:

| | |
|---|---|
| 1. Eingangstemperatur | 50 °C |
| 2. Sprühflow | 800 NL/h |

Die Mikropartikel fallen als weißes, rieselfähiges Pulver an. Ausbeute: 1,2 g = 60 % der Theorie.

| | |
|---|---|
| Wirkstoffgehalt (Beladungsgrad): | 3 % |
| Restlösungsmittelgehalt: Eisessig | 0,8 % |

b) In gleicher Weise werden Mikropartikel hergestellt unter Verwendung von 0,192 g Buserelinacetat und 1,808 g PLGA 50:50 (IV = 0,4 dl/g). Ausbeute: 1,2 g = 60 % der Theorie.

| | |
|---|---|
| Wirkstoffgehalt: | 9 % |
| Restlösungsmittelgehalt: Eisessig | 0,8 % |

Für 20 mg Mikropartikel wurden die in vitro-Freigaberaten wie folgt bestimmt:
20 mg Mikropartikel werden in 2,0 ml einer Phosphatpufferlösung pH 7,4 eingebracht und 24 h lang bei +37 °C eluiert. Nach Trennung des Eluates von den Mikropartikel wird das Eluat mit Hilfe eines HPLC-Verfahrens auf Buserelingehalt geprüft. Die zurückbleibenden Mikropartikel werden erneut in 2,0 ml Phosphatpuffer gegeben und wiederum 24 h lang bei 37 °C eluiert. Dieser Prozeß wird über 56 Tage analog fortgesetzt.

Die aus den Eluaten bestimmten Buserelinacetat-Mengen sind in der folgenden Tabelle ausschnittsweise zusammengestellt:

| | Wirkstoffgehalt 3% | Wirkstoffgehalt 9% |
|---|---|---|
| Tag 2 | 5,8 »g | 23,2 »g |
| Tag 9 | <0,7 »g | 57,7 »g |
| Tag 16 | 13,4 »g | 51,9 »g |
| Tag 23 | 20,3 »g | 16,7 »g |
| Tag 30 | 13,2 »g | 8,4 »g |
| Tag 37 | 4,1 »g | 3,4 »g |
| Tag 44 | 2,5 »g | 1,5 »g |
| Tag 51 | 0,7 »g | 0,7 »g |
| Tag 56 | 0,7 »g | 0,7 »g |

### Beispiel 2: Sprühmedium - Chloroform

a) 0,108 g Buserelinacetat werden in 2,0 g Wasser gelöst. Die wäßrige Lösung wird mit Hilfe eines Rotor-Stator-Homogenisators in einer Lösung von 1,892 g PLGA 50:50 (IV=0,4dl/g) in 92 g Chloroform emulgiert und in einem Laborsprühtrockner versprüht.
Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 0,9 g = 45 % der Theorie.

| | |
|---|---|
| Wirkstoffgehalt: | 5 % |

b) In gleicher Weise werden Mikropartikel hergestellt, unter Verwendung von 0,152 g Buserelinacetat und 1,848 g PLGA 50:50 (IV = 0,4dl/g). Ausbeute: 1 g = 50% der Theorie.

| | |
|---|---|
| Wirkstoffgehalt: | 7 % |

Für 20 mg Mikropartikel wurden die in vitro-Freigaberaten analog Beispiel 1 über 35 Tage bestimmt:
Die aus den Eluaten bestimmten Buserelinacetat-Mengen sind in der folgenden Tabelle ausschnittweise zusammengestellt:

| | Wirkstoffgehalt 5 % | Wirkstoffgehalt 7 % |
|---|---|---|
| Tag 2 | 44,7 »g | 56,2 »g |
| Tag 9 | 7,3 »g | 10,1 »g |
| Tag 16 | 30,3 »g | 37,8 »g |
| Tag 23 | 30,0 »g | 38,6 »g |
| Tag 30 | 10,9 »g | 13,6 »g |
| Tag 35 | 11,5 »g | 14,5 »g |

### Beipiel 3: Sprühmedium - Methylenchlorid/Wasser/Methanol

a) 0,128 g Buserelinacetat werden in 2,0 g Wasser gelöst. Die wäßrige Lösung wird mit einer Lösung von 1,872 g PLGA 50:50 (IV =0,4dl/g) in 46 g Methylenchlorid gemischt. Zu der Mischung werden 9 g Methanol hinzugegeben. Es entsteht eine klare Lösung, die in einem Laborsprühtrockner versprüht wird.

Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 1,2 g = 60 % der Theorie.

| | |
|---|---|
| Wirkstoffgehalt: | 6 % |

| | | |
|---|---|---|
| Restlösungsmittelgehalt: | Wasser | 0,9 % |
| | Methylenchlorid | 0,06 % |
| | Methanol | <0,01 % |

Für 20 mg Mikropartikel wurden die in vitro-Freigaberaten analog Beispiel 1 über 28 Tage bestimmt:
Die aus den Eluaten bestimmte Buserelinacetat-Mengen sind in der folgenden Tabelle ausschnittweise zusammengestellt:

| | Wirkstoffgehalt 6 % |
|---|---|
| Tag 2 | 93,7 »g |
| Tag 9 | 5,1 »g |
| Tag 16 | 7,4 »g |
| Tag 23 | 12,1 »g |
| Tag 28 | 12,1 »g |

### Beispiel 4: Sprühmedium - Methylenchlorid/Wasser/Methanol

a) 0,084 g Buserelinacetat werden in 4,0 g Wasser gelöst. Die wäßrige Lösung wird mit einer Lösung von 1,916 g PLGA 50:50 (IV=0,42dl/g) in 65 ml Methylenchlorid gemischt. Zu der Mischung werden 25 g Methanol hinzugegeben. Es entsteht eine klare Lösung, die in einem Laborsprühtrockner versprüht wird.
Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 0,8 g = 40 % der Theorie.

| | |
|---|---|
| Wirkstoffgehalt: | 4 % |

b) In gleicher Weise werden Mikropartikel hergestellt, unter Verwendung von 0,168 g Buserelinacetat und 1,832 g PLGA 50:50 (IV = 0,42 dl/g). Ausbeute: 0,8 g = 40 % der Theorie.

| | |
|---|---|
| Wirkstoffgehalt: | 8 % |

Für 20 mg Mikropartikel wurden die in vitro-Freigaberaten wie folgt bestimmt:
20 mg Mikropartikel werden in 2,0 ml einer Phosphatpufferlösung pH 7,4 eingebracht und 24 h lang bei +37°C eluiert. Nach Trennung des Eluates von den Mikropartikel wird das Eluat mit Hilfe eines Radioimmunoassays (RIA) auf Buserelingehalt geprüft. Die zurückbleibenden Mikropartikel werden erneut in 2,0 ml Phosphatpuffer gegeben und wiederum 24 h lang bei 37°C eluiert.
Dieser Prozeß wird über 63 Tage analog fortgesetzt.
Die aus den Eluaten bestimmten Buserelinacetat-Mengen sind in der folgenden Tabelle ausschnittweise zusammengestellt:

| | Wirkstoffgehalt 4 % | Wirkstoffgehalt 8 % |
|---|---|---|
| Tag 2 | 129,3 »g | 280,1 »g |
| Tag 9 | 1,7 »g | 4,7 »g |
| Tag 16 | 1,9 »g | 5,7 »g |
| Tag 23 | 6,3 »g | 3,0 »g |
| Tag 30 | 6,4 »g | 8,1 »g |
| Tag 37 | 3,1 »g | 5,6 »g |
| Tag 44 | 1,6 »g | 3,9 »g |
| Tag 51 | 3,1 »g | 2,4 »g |
| Tag 58 | 0,7 »g | 1,4 »g |
| Tag 63 | 0,7 »g | 1,4 »g |

### Beispiel 5: Sprühmedium - Chloroform/Wasser/Dimethylformamid

a) 0,13 g Buserelinacetat werden in 2,0 g Wasser gelöst. Die wäßrige Lösung wird mit Hilfe eines Rotor-Stator-Homogenisators in einer Lösung von 1,87 g PLGA 50:50 (IV=0,4dl/g) in 41,4g Chloroform und 4,6 g Dimethylformamid emulgiert und in einem Laborsprühtrockner versprüht.

Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 1,2 g = 60 % der Theorie.

| | |
|---|---|
| Wirkstoffgehalt: | 6 % |

Für 20 mg Mikropartikel wurden die in vitro-Freigaberaten analog Beispiel 4 über 63 Tage bestimmt:
Die aus den Eluaten bestimmten Buserelinacetat-Mengen sind in der folgenden Tabelle ausschnittweise zusammengestellt:

| | Wirkstoffgehalt 6 % |
|---|---|
| Tag 2 | 32,1 »g |
| Tag 9 | 16,8 »g |
| Tag 16 | 49,1 »g |
| Tag 23 | 39,1 »g |
| Tag 30 | 17,5 »g |
| Tag 37 | 5,7 »g |
| Tag 44 | 2,5 »g |
| Tag 51 | 2,5 »g |
| Tag 58 | 1,4 »g |
| Tag 63 | 1,4 »g |

### Beispiel 6: Sprühmedium - Methylenchlorid

a) 0,085 g Buserelinacetat werden in 2,0 g Wasser gelöst. Die wäßrige Lösung wird mit Hilfe eines Rotor-Stator-Homogenisators in einer Lösung von 1,915 g PLGA 50:50 (IV=0,4dl/g) in 46 g Methylenchlorid emulgiert und in einem Laborsprühtrockner versprüht.
Die Sprühparameter werden wie folgt eingestelt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 0,6 g = 30 % der Theorie.

| | |
|---|---|
| Wirkstoffgehalt: | 4 % |

b) In gleicher Weise werden Mikropartikel hergestellt, unter Verwendung von 0,128 g Buserelinacetat und 1,872 g PLGA 50:50 (IV = 0,4dl/g), Ausbeute: 0,7 g = 35 % der Theorie.

| | |
|---|---|
| Wirkstoffgehalt: | 6 % |

c) sowie unter Verwendung von 0,170 g Buserelinacetat und 1,872 g PLGA 50:50 (IV=0,4dl/g).
Ausbeute: 0,4 g = 20 % der Theorie.

| | |
|---|---|
| Wirkstoffgehalt: | 8 % |

Für 20 mg der Mikropartikel wurden die in vitro-Freigaberaten analog Beispiel 4 über 63 Tage bestimmt:
Die aus den Eluaten bestimmten Buserelinacetat-Mengen sind in der folgenden Tabelle ausschnittweise zusammengestellt:

| | Wirkstoffgehalt 4 % | Wirkstoffgehalt 6 % | Wirkstoffgehalt 8 % |
|---|---|---|---|
| Tag 2 | 39,4 »g | 49,8 »g | 88,0 »g |
| Tag 9 | 1,1 »g | 2,7 »g | 4,7 »g |
| Tag 16 | 5,9 »g | 34,3 »g | 21,7 »g |
| Tag 23 | 17,1 »g | 51,5 »g | 28,0 »g |
| Tag 30 | 4,2 »g | 18,4 »g | 3,2 »g |
| Tag 37 | 1,5 »g | 5,8 »g | 2,2 »g |
| Tag 44 | 1,7 »g | 2,5 »g | 2,8 »g |
| Tag 51 | 0,4 »g | 0,8 »g | 2,4 »g |
| Tag 58 | 0,3 »g | 0,7 »g | 2,5 »g |
| Tag 63 | 0,3 »g | 0,3 »g | 2,5 »g |

### Beispiel 7: Sprühmedium - Methylenchlorid/Wasser

0,032 g Buserelinacetat werden in 1,0 g Wasser gelöst. Die wäßrige Lösung wird mit Hilfe eines Rotor-Stator-Homogenisators in einer Lösung von 0,968 g PLGA 85:15 in 23 g Methylenchlorid emulgiert und in einem Laborsprühtrockner versprüht.
Die Sprühparameter werden wie folgt eingestellt:

| | |
|---|---|
| 1. Eingangstemperatur | 40°C |
| 2. Sprühflow | 800 NL/h |

Die Mikropartikel fallen als weißes, rieselfähiges Pulver an. Ausbeute: 0,7 g = 70 % der Theorie.

| | |
|---|---|
| Wirkstoffgehalt: | 3 % |

### Beispiel 8: Sprühmedium - Methylenchlorid/Wasser

0,064 g Buserelinacetat werden in 2,0 g Wasser gelöst. Die wäßrige Lösung wird mit Hilfe eines Rotor-Stator-Homogenisators in einer Lösung von 1,936 g PLGA 50:50 (IV=0,7dl/g) in 92 g Methylenchlorid emulgiert und in einem Laborsprühtrockner versprüht.
Die Sprühparameter werden wie folgt eingestellt:

| | |
|---|---|
| 1. Eingangstemperatur | 40°C |
| 2. Sprühflow | 800 NL/h |

Die Mikropartikel fallen als weißes, rieselfähiges Pulver an, Ausbeute: 0,9 g = 45 % der Theorie.

| | |
|---|---|
| Wirkstoffgehalt: | 3 % |

Bestimmung der Wirkstofffreigabe analog Beispiel 1

| | Wirkstoffgehalt 3 % |
|---|---|
| Tag 2 | 48,1 »g |
| Tag 23 | 3,9 »g |
| Tag 37 | 7,1 »g |
| Tag 44 | 3,9 »g |
| Tag 51 | 1,0 »g |

### Beispiel 9: Sprühmedium - Methylenchlorid/Wasser

0,106 g Buserelinacetat werden in 1,0 g Wasser gelöst. Die wäßrige Lösung wird mit Hilfe eines Rotor-Stator-Homogenisators in einer Lösung von 0,894 g PLGA 50:50 (IV=0,1dl/g) in 23 g Methylenchlorid emulgiert und in einem Laborsprühtrockner versprüht.

Die Sprühparameter werden wie folgt eingestellt:

| | |
|---|---|
| 1. Eingangstemperatur | 40°C |
| 2. Sprühflow | 800 NL/h |

Die Mikropartikel fallen als weißes, rieselfähiges Pulver an. Ausbeute: 0,3 g = 30 % der Theorie.

| | |
|---|---|
| Wirkstoffgehalt: | 10 % |

Bestimmung der Wirkstoffeingabe analog Beispiel 1

| | Wirkstoffgehalt 10 % |
|---|---|
| Tag 2 | 32,7 »g |
| Tag 9 | 19,6 »g |
| Tag 16 | 20,6 »g |
| Tag 23 | 15,4 »g |
| Tag 28 | 15,4 »g |

### Beispiel 10: Sprühmedium - Methylenchlorid/Wasser

0,13 g Buserelinacetat werden in 2,0 g Wasser gelöst. Die wäßrige Lösung wird mit Hilfe eines Rotor-Stator-Homogenisators in einer Lösung von 1,87 g PLGA 75:25 (IV=0,5dl/g) in 46 g Methylenchlorid emulgiert und in einem Laborsprühtrockner versprüht.
Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 1,2 g = 60 % der Theorie.

| | |
|---|---|
| Wirkstoffgehalt: | 6 % |

### Beispiel 11: Sprühmedium - Methylenchlorid/Wasser

0,128 g Buserelinacetat werden in 2,0 g Wasser gelöst. Die wäßrige Lösung wird mit Hilfe eines Rotor-Stator-Homogenisators in einer Lösung von 1,872 g PLGA 75:25 (IV=0,8dl/g) in 138 g Methylenchlorid emulgiert und in einem Laborsprühtrockener versprüht.
Die Sprühparameter werden wie folgt eingestellt:

| | |
|---|---|
| 1. Eingangstemperatur: | 30°C |
| 2. Sprühflow | 800 NL/h |

Die Mikropartikel fallen als weißes, rieselfähiges Pulver an. Ausbeute: 0,9 g = 45 % der Theorie.

| | |
|---|---|
| Wirkstoffgehalt: | 6 % |

### Beispiel 12: Sprühmedium - Methylenchlorid/Wasser

0,13 g Buserelinacetat werden in 2,0 g Wasser gelöst. Die wäßrige Lösung wird mit Hilfe eines Rotor-Stator-Homogenisators in einer Lösung von 1,87 g PLGA 47:53 (IV=0,3dl/g) in 46 g Methylenchlorid emulgiert und in einem Laborsprühtrockner versprüht.
Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an, Ausbeute: 1,3 g = 65 % der Theorie.

| | |
|---|---|
| Wirkstoffgehalt: | 6 % |

### Beispiel 13: Sprühmedium - Methylenchlorid

0,13 g Buserelinacetat werden in einer Lösung aus 1,87 g PLGA 50:50 (IV=0,4dl/g) und 46 g Methylenchlorid mit Hilfe eines Rotor-Stator-Homogenisators suspendiert. Die Suspension wird in einem Laborsprühtrockner versprüht.
Die Sprühparameter werden wie folgt eingestellt:

| | |
|---|---|
| 1. Eingangstemperatur | 40°C |
| 2. Sprühflow | 800 NL/h |

Die Mikropartikel fallen als weißes, rieselfähiges Pulver an. Ausbeute: 0,5 g = 25 % der Theorie.

| | |
|---|---|
| Wirkstoffgehalt: | 6 % |

### Beispiel 14: Sprühmedium - Methylenchlorid/Wasser

a) 0,2 g Hoe 427 werden in 1,0 g Wasser gelöst. Die wäßrige Lösung wird mit Hilfe eines Rotor-Stator-Homogenisators in einer Lösung von 3,8 g PLGA 50:50 (IV=0,4dl/g) in 92 g Methylenchlorid emulgiert und in einem Laborsprühtrockner versprüht.
Die Sprühparameter werden wie folgt eingestellt:

| | |
|---|---|
| 1. Eingangstemperatur | 40°C |
| 2. Sprühflow | 800 NL/h |

Die Mikropartikel fallen als weißes, rieselfähiges Pulver an. Ausbeute: 1,9 g = 48 % der Theorie.

| | |
|---|---|
| Wirkstoffgehalt: | 5 % |

b) In gleicher Weise werden Mikropartikel hergestellt, unter Verwendung von 0,6 g Hoe 427 und 3,4 g PLGA 50:50 (IV=0,4dl/g), Ausbeute: 2,8 g = 70 % der Theorie.

| | |
|---|---|
| Wirkstoffgehalt: | 15 % |

c) sowie unter Verwendung von 0,4 g Hoe 427 und 3,6 g PLGA 50:50 (IV=0,4dl/g). Ausbeute: 2 g = 50 % der Theorie.

| | |
|---|---|
| Wirkstoffgehalt: | 10 % |

### Beispiel 15: Sprühmedium - Methylenchlorid/Wasser

0,08 g Hoe 013 werden in 1,0 g Wasser gelöst. Die wäßrige Lösung wird mit Hilfe eines Rotor-Stator-Homogenisators in einer Lösung von 0,92 g PLGA 50:50 (IV=0,4dl/g) in 23 g Methylenchlorid emulgiert und in einem Laborsprühtrockner versprüht.

Die Sprühparameter werden wie folgt eingestellt:

| | |
|---|---|
| 1. Eingangstemperatur | 30°C |
| 2. Sprühflow | 800 NL/h |

Die Mikropartikel fallen als weißes, rieselfähiges Pulver an. Ausbeute: 0,5 g = 50 % der Theorie.

| | |
|---|---|
| Wirkstoffgehalt: | 6 % |

Bestimmung der Wirkstofffreigabe analog Beispiel 1

| | Wirkstoffgehalt 6 % |
|---|---|
| Tag 2 | 13,9 »g |
| Tag 9 | 5,9 »g |
| Tag 16 | 3,1 »g |
| Tag 23 | 3,7 »g |
| Tag 28 | 2,2 »g |

## Patentansprüche

1. Verfahren zur Herstellung von langwirkenden bioabbaubaren Mikropartikeln, die ein therapeutische wirksames Peptid als Wirkstoff und Poly(lactid-co-glycolid) (PLGA) als Trägermaterial enthalten, dadurch gekennzeichnet, daß man das Peptid in einer Lösung des Trägermaterials suspendiert oder löst oder eine wäßrige Lösung des Peptids in einer Lösung des Trägermaterials emulgiert und die Suspension, Lösung oder Emulsion bei einer Temperatur unter 60 °C bei einem Sprühflow größer als 500 L/h (500 NL/h) versprüht.

2. Verfahren gemäß Anspruch 1, dadurch gekennzeichnet, daß das Peptide ein wasserlösliches Peptid ist.

3. Verfahren gemäß Anspruch 1, dadurch gekennzeichnet, daß eine oder mehrere der folgenden Bedingungen eingehalten werden:
a) das Peptid ein LHRH-Agonist oder LHRH-Antagonist ist
b) das Trägermaterial PLGA 50:50 ist
c) die inhärente Viskosität des PLGA 0,1 bis 0,5 dl/g (20 °C, Chloroform, 0,1 %) ist
d) das Lösungsmittel Chloroform, Methylenchlorid, Eisessig, Methanol, Dimethylformamid oder Wasser oder ein Gemisch aus mindestens 2 der genannten Lösungsmittel ist und
e) der Sprühflow etwa 800 L/h (800 NL/h) beträgt.

4. Verfahren gemäß Anspruch 1, dadurch gekennzeichnet, daß eine oder mehrere der folgenden Bedingungen eingehalten werden:
a) das Peptid Buserelinacetat oder HOE 013 ist
b) das Trägermaterial PLGA 50:50 ist
c) die inhärente Viskosität des PLGA 0,1 bis 0,5 dl/g (20°C, Chloroform, 0,1 %) ist
d) das Lösungsmittel Methylenchlorid, Eisessig oder Wasser bzw. ein Gemisch aus 2 der genannten Lösungsmittel ist und
e) der Sprühflow etwa 800 L/h (= 800 NL/h beträgt).

## Claims

1. A process for the preparation of long-acting biodegradable microparticles which contain a therapeutically active peptide as the active substance and poly(lactide-co-glycolide) (PLGA) as the support material, which comprises suspending or dissolving the peptide in a solution of the support material or emulsifying an aqueous solution of the peptide in a solution of the support material and spraying the suspension, solution or emulsion at a temperature below 60°C at a spray flow rate greater than 500 L/h (500 NL/h).

2. The process as claimed in claim 1, wherein the peptide is a water-soluble peptide.

3. The process as claimed in claim 1, wherein one or more of the following conditions are kept to:
a) the peptide is an LHRH agonist or LHRH antagonist
b) the support material is 50:50 PLGA
c) the inherent viscosity of the PLGA is 0.1 to 0.5 dl/g (20°C, chloroform, 0.1%)
d) the solvent is chloroform, methylene chloride, glacial acetic acid, methanol, dimethylformamide or water or a mixture of at least 2 of the solvents mentioned and
e) the spray flow rate is about 800 L/h (800 NL/h).

4. The process as claimed in claim 1, wherein one or more of the following conditions are kept to:
a) the peptide is buserelin acetate or HOE 013
b) the support material is 50:50 PLGA
c) the inherent viscosity of the PLGA is 0.1 to 0.5 dl/g (20°C, chloroform, 0.1%)
d) the solvent is methylene chloride, glacial acetic acid or water, or a mixture of 2 of the solvents mentioned and
e) the spray flow rate is about 800 L/h (= 800 NL/h).

## Revendications

1. Procédé pour la préparation de microparticules biodégradables, à longue durée d'action, qui contiennent un peptide à activité thérapeutique en tant que substance active et un poly(lactide-co-glycolide) (PLGA) en tant que substance véhicule, caractérisé en ce que l'on dissout ou met en suspension le peptide dans une solution de la substance véhicule, ou on émulsionne une solution aqueuse du peptide dans une solution de la substance véhicule, et on pulvérise la suspension, solution ou émulsion à une température inférieure à 60°C et à un débit de pulvérisation supérieur à 500 l/h (500 litres normaux/h).

2. Procédé selon la revendication 1, caractérisé en ce que le peptide est un peptide soluble dans l'eau.

3. Procédé selon la revendication 1, caractérisé en ce que l'on maintient une ou plusieurs des conditions suivantes:
a) le peptide est un agoniste de LHRH ou un antagoniste de LHRH,
b) la substance véhicule est le PLGA 50:50,
c) la viscosité inhérente du PLGA va de 0,1 à 0,5 dl/g (à 20°C, chloroforme, 0,1 %),
d) le solvant est le chloroforme, le chlorure de méthylène, l'acide acétique glacial, le méthanol, le diméthylformamide ou l'eau, ou un mélange d'au moins deux des solvants cités, et
e) le débit de pulvérisation est d'environ 800 l/h (800 ln/h).

4. Procédé selon la revendication 1, caractérisé en ce que l'on maintient une ou plusieurs des conditions suivantes:
a) le peptide est la buséréline acétate ou HOE 013,
b) la substance véhicule est le PLGA 50:50,
c) la viscosité inhérente du PLGA va de 0,1 à 0,5 dl/g (à 20°C, chloroforme, 0,1 %),
d) le solvant est le chlorure de méthylène, l'acide acétique glacial ou l'eau, ou un mélange de deux des solvants cités, et
e) le débit de pulvérisation est d'environ 800 l/h (800 ln/h).
